# NEUE EUROPÄISCHE PATENTSCHRIFT

(11) **EP 0 692 268 B2**
(45) Veröffentlichungstag und Bekanntmachung des Hinweises auf die Entscheidung über den Einspruch: **09.02.2005**
(45) Hinweis auf die Patenterteilung: 13.10.1999
(21) Anmeldenummer: 95110612.9
(22) Anmeldetag: 07.07.1995
(51) Int. Cl.: A61M 1/34

(54) **Substituataufbereitung bei der Hämofiltration oder Hämodiafiltration**
Preparation of substitution liquid in a haemofiltration apparatus or haemodiafiltration apparatus
Préparation du liquide de substitution dans un appareil d'hémofiltration ou d'hémodiafiltration

(30) Priorität: 13.07.1994 DE 4424692
(43) Veröffentlichungstag der Anmeldung: 17.01.1996
(73) Patentinhaber: Fresenius Medical Care Deutschland GmbH, 61352 Bad Homburg (DE)
(72) Erfinder: Nederlof, Bernd, D-66606 St. Wendel (DE)
(74) Vertreter: Luderschmidt, Schüler & Partner GbR

(56) Entgegenhaltungen:
- EP-A- 0 516 152
- EP-A- 0 622 087
- EP-B- 0 270 794
- EP-B- 0 571 303
- DE-A- 3 444 671
- US-A- 4 206 055
- US-A- 4 708 802
- US-A- 4 784 768
- Artikel "Filtration of dialysate using on-line dialysate filter", S. Frinak et al., The International Journal of Artificial Organs, vol. 14, no. 11, 1991, Seiten 691-697
- Artikel "On-line preparation of sterile pyrogen-fee electrolyte solution", L.W. Henderson et al., Vol. XXIV Trans. Am. Soc. Artif. Intern. Organs, 1978, Seiten 465-467

## Beschreibung

Die Erfindung betrifft eine Hämofiltrationsvorrichtung oder Hämodiafiltrationsvorrichtung mit einem Hämofilter bzw. Dialysator, der durch eine Membran in zwei Kammern geteilt ist, wobei die erste Kammer Teil eines Dialysierflüssigkeitsweges und die zweite Kammer Teil eines Blutweges ist, der Dialysierflüssigkeitsweg eine Zuleitung, die sich von einer Einrichtung zur Bereitstellung von Dialysierflüssigkeit bis zum Hämofilter bzw. Dialysator erstreckt und in die eine erste Bilanziereinheit geschaltet ist, und eine Ableitung aufweist, die sich vom Hämofilter bzw. Dialysator zu einem Abfluß erstreckt und in die eine zweite Bilanziereinheit geschaltet ist, mit einer Pumpe zum Fördern der Dialysierflüssigkeit im geschlossenen Dialysierflüssigkeitssystem, einer die Zuleitung mit der Ableitung des Dialysierflüssigkeitsweges verbindenden Bypassleitung, in die ein Bypassventil geschaltet ist, einer Ultrafiltrationseinrichtung, einem in der Zuleitung zwischen der ersten Bilanziereinheit und dem Hämofilter bzw. Dialysator angeordneten ersten Sterilfilter, der durch eine Keime zurückhaltende Membran in eine erste Kammer und eine zweite Kammer geteilt ist, einer von der Zuleitung zwischen der ersten Bilanziereinheit und dem Hämofilter bzw. Dialysator abgehenden Verbindungsleitung, die mit dem Blutweg verbunden ist und in die eine Pumpe und mindestens ein zweiter Sterilfilter, der durch eine Keime zurückhaltende Membran in eine erste Kammer und eine zweite Kammer geteilt ist, geschaltet sind.

Bei der Hämofiltration wird -ähnlich wie bei der Hämodialyse - Blut an der Membran eines Hämofilters vorbeigeleitet, wobei ein Teil des Serums durch die Membran abgezogen und durch eine sterile Substitutionsflüssigkeit ersetzt wird, die entweder stromauf des Dialysators (Prädilution) oder stromab des Dialysators (Postdilution) dem extrakorporalen Blutweg zugesetzt wird. Zusätzlich wird bei der Hämodiafiltration noch die übliche Hämodialyse durchgeführt, d.h., es wird an der Membran des Hämodialysators Dialysierflüssigkeit vorbeigeleitet, so daß über die Membran hinweg ein Austausch von harnpflichtigen Substanzen erfolgen kann.

Die Dialysierflüssigkeit kann on-line aus Frischwasser und einem Elektrolytkonzentrat und die Substitutionsflüssigkeit on-line aus der Dialysierflüssigkeit hergestellt werden. Das Elektrolytkonzentrat ist zwar in der Regel steril und das Frischwasser weist üblicherweise keine Keime auf, es ist jedoch nicht sichergestellt, daß die on-line hergestellte Dialysierflüssigkeit absolut steril und pyrogenfrei ist, weshalb die Dialysierflüssigkeit zur Herstellung der Substitutionsflüssigkeit in den sterilen und pyrogenfreien Zustand überführt wird. Dazu wird stromauf des Dialysators mit einer Leitung Dialysierflüssigkeit entnommen, wobei in diese Leitung mindestens ein Sterilfilter eingeschaltet ist.

Eine derartige Vorrichtung ist beispielsweise aus der DE 34 44 671 A oder der EP 0 042 939 bekannt.

Diese genannten Vorrichtungen haben jedoch durch die sog. "Dead-end"-Anordnung des Sterilfilters in der Substituatleitung den Nachteil, daß sich im Laute der Zeit Partikel und andere Substanzen, z.B. eingeschleppt Keime und Pyrogene vor der Membran des Sterilfilters ansammeln. Dies ist insbesondere dann gefährlich, wenn durch eine Ruptur diese Substanzen schlagartig in den Sterilbereich eingeschleppt werden und die Substitutionsflüssigkeit damit kontaminiert wird.

Der Erfindung liegt daher die Aufgabe zugrunde, eine Hämofiltrationsvorrichtung oder Hämodiafiltrationsvorrichtung der eingangs genannten Art so fortzubilden, daß eine Ansammlung mit Keimen oder Pyrogenen im Sterilfilter Weitgehend verhindert wird, die Sicherheit der Hämofiltrationrvorrichtung oder Hämodiafiltrationsvorrichtung deutlich verbessert und die Sterilität der Dialysierflüssigkeit und des Substituats durch Vermeidung von Rupturen gewährleistet ist.

Die Lösung der Aufgabe erfolgt mit den Merkmalen des Anspruchs 1.

Durch diese erfindungsgemäBe Anordnung des zweiten Steriffilters ist es möglich, die Dialysierflüssigkeit durch die erste Kammer hindurchströmen zu lassen und damit die Membran des zweiten Sterilfilters freizuspülen, so daß eine Ansammlung von Keimen oder Pyrogenen vor den Poren der Membran verhindert wird.

Der Auslaß der ersten Kammer des zweiten Sterilfilters ist mit dem Eingang der ersten Kammer des Dialysators verbunden. Durch diese erfindungsgemäße Anordnung des zweiten Sterilfilters wird zum einen die als Substituatlösung abgezweigte Dialysierflüssigkeit einer zweiten, sterilisierend wirkenden Filtration unterzogen, so daß auch bei einer eventuellen Undichtigkeit der Membran des ersten Sterilfilters eine Kontamination der Substituatlösung zuverlässig verhindert wird. Zum anderen wird durch diese erfindungsgemäße Anordnung die Membran des zweiten Sterilfilters durch die durch die erste Kammer hindurchströmende Dialysierflüssigkeit ständig freigespült, so daß eine Ansammlung von eingeschleppten Keimen und Pyrogenen zuverlässig verhindert wird. Damit wird auch hier die Gefahr einer Ruptur deutlich reduziert, so daß die Sterilität der Substituatlösung gewährleistet und die Sicherheit der Hämodiafiltrationsvorrichtung deutlich verbessert ist.

Eine bevorzugte Weiterbildung der erfindungsgemäßen Hämofiltrationsvorrichtung oder Hämodiafiltrationsvorrichtung sieht vor, den ersten Sterilfilter in die Zuleitung einzuschalten und die erste Kammer des ersten Sterilfilters ebenfalls zumindest zeitweise in Durchfluß zu schalten.

Gemäß einer vorteilhaften Ausbildung wird dazu die erste Kammer des ersten Sterilfilters mit der zur Ableitung führenden Bypassleitung verbunden.

Durch diese Anordnung des ersten Sterilfilters erfolgt eine sterilisierend wirkende Filtration der Dialysierflüssigkeit, so daß dem nachgeschalteten zweiten Sterilfilter eine absolut sterile und pyrogenfreie Dialysierflüssigkeit zugeführt wird. Durch Öffnen des Bypassventils, welches sowohl während der Behandlung als auch während des Spülbetriebs der gesamten Anordnung geöffnet werden kann, fließt die Dialysierflüssigkeit aus der ersten Kammer des Sterilfilters ab und reißt dort an der Membran befindliche Pyrogene und Teilchen in die Ableitung und von dort in den Abfluß mit. Das Freispülen der Membran kann dabei in vorbestimmten Abständen erfolgen, so daß das Ansammeln von Keimen und Pyrogenen zuverlässig verhindert wird. Da die Anhäufung vor der Membran mit der daraus resultierenden Erhöhung des Transmembrandrucks (TMP) ein Hauptgrund für das Entstehen einer Ruptur ist, wird das Entstehen einer Ruptur somit unwahrscheinlicher, so daß die Sterilität der Dialyserflüssigkeit gewährleistet ist und die Sicherheit der Hämodiafiltrationsvorrichtung verbessert wird.

Insgesamt wird somit eine Hamofiltrationsvorrichtung oder Hämodiafiltrationsvorrichtung bereitgestellt, die sowohl die Kontamination des Blutes im Dialysator als auch die Kontamination der Substituatlösung durch eingeschleppte Keime oder Pyrogene mit einer zweifachen Sicherheit verhindert. Des weiteren wird zuverlässig die Ansammlung von Pyrogenen verhindert, so daß eine Hämofiltrationsvorrichtung oder Hämodiafiltrationsvorrichhing mit einem hohen Sicherheitsniveau bereitgestellt wird.

Unter "Bilanziervorrichtung" werden erfindungsgemäß solche Systeme verstanden, mit denen zu- und abgeförderte Flüssigkeitsmengen exakt volumetrisch gegeneinander bestimmt (bilanziert) werden können. Solche Bilanziervorrichtungen sind beispielsweise aus der DE 28 38 414 bekannt, worauf Bezug genommen wird, und enthalten als Bilanziereinheiten üblicherweise Bilanziereinheiten mit einem definierten Innenvolumen. Andererseits werden erfindungsgemäß von der Bilanziereinheit auch andere Einrichtungen verstanden, die bilanzmäßig arbeiten, beispielsweise bilanzierende Pumpen und dergleichen.

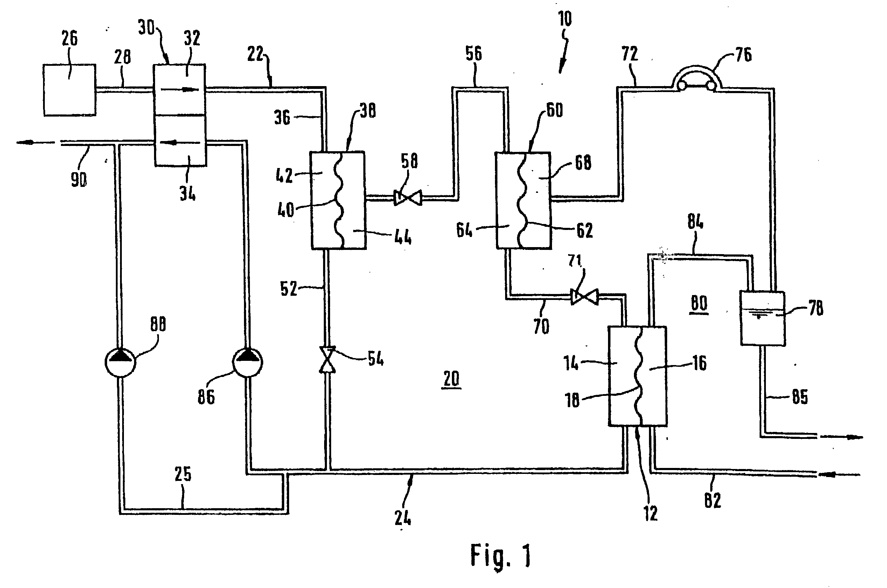

Weitere Einzelheiten, Merkmale und Vorteile der Erfindung sind anhand der nachfolgenden Beschreibung eines Ausführungsbeispieles unter Bezugnahme auf die Zeichnung näher erläutert, in der
Fig. 1 ein erstes Ausführungsbeispiel einer Hämodiafiltrationsvorrichtung in einer schematischen Darstellung zeigt.

In Fig. 1 ist mit 10 eine Hämodiafiltrationsvorrichtung gezeigt, die einen üblichen Dialysator 12 aufweist, der durch eine Membran 18 in eine von Dialysierflüssigkeit durchflossene erste Kammer 14 und eine von Blut durchflossene zweite Kammer 16 getrennt ist

Die erste Kammer 14 ist in einen Dialysierflüssigkeitsweg 20 eingeschaltet, der aus einer Zuleitung 22 und einer Ableitung 24 besteht, und die zweite Kammer 16 ist in einen Blutweg 80 eingeschaltet.

Die Zuleitung 22 besteht aus einem ersten Zuleitungsabschnitt 28, einem zweiten Zuleitungsabschnitt 36, einem dritten Zuleitungsabschnitt 56 und einem vierten Zuleitungsabschnitt 70 und verbindet eine Dialysierflüssigkeitsquelle 26 mit der ersten Kammer 14 des Dialysators 12.

Der erste Zuleitungsabschnitt 28 verbindet die Dialysierflüssigkeitsquelle 26 mit der ersten Kammer (Bilanziereinheit) 32 einer Bilanzierungsvorrichtung 30. Die erste Kammer 32 der Bilanzierungsvorrichtung 30 ist über den zweiten Zuleitungsabschnitt 36 mit der ersten Kammer 42 eines ersten Sterilfilters 38 verbunden. Der erste Sterilfilter 38 ist durch eine Membran 40 in eine erste Kammer 42 und eine zweite Kammer 44 unterteilt. Der Ausgang der ersten Kammer 42 des ersten Sterilfilters 38 ist mit einer Bypassleitung 52 verbunden, in die ein Bypassventil 54 eingeschaltet ist und die mit der Ableitung 24 verbunden ist

Von der zweiten Kammer 44 des ersten Sterilfilters 38 geht der dritte Zuleitungsabschnitt 56 ab und ist mit der ersten Kammer 64 eines zweiten Sterilfilters 60 verbunden. In dem dritten Zuleitungsabschnitt 56 ist ein Ventil 58 eingeschaltet. Der zweite Sterilfilter 60 wird durch eine Membran 62 in eine erste Kammer 64 und eine zweite Kammer 68 unterteilt. Der Ausgang der ersten Kammer 64 des zweiten Sterilfilters 60 ist mit dem vierten Zuleitungsabschnitt 70 verbunden, welcher zur ersten Kammer 14 des Dialysators 12 geführt ist.

Vom Ausgang der ersten Kammer 14 des Dialysators 12 führt die Ableitung 24 zur zweiten Bilanziereinheit 34 der Bilanzierungsvorrichtung 30. In der Ableitung 24 ist eine Dialysierflüssigkeitspumpe 86 eingeschaltet, des weiteren geht von der Ableitung 24 eine Ultrafiltratleitung 25 ab, in die eine Ultrafiltrationspumpe 88 eingeschaltet ist. Die Ultrafiltratleitung 25 führt zu einem Abfluß 90, an den auch der Ausgang der zweiten Bilanziereinheit 34 der Bilanzierungsvorrichtung 30 angeschlossen ist.

Die zweite Kammer 16 des Dialysators 12 ist dergestalt in den Blutweg eingeschaltet, daß eine vom Patienten kommende Blutzleitung 82 an den Eingang der zweiten Kammer 16 angeschlossen ist und ein erster Abschnitt 84 einer Blutableitung an den Ausgang der Kammer 16 angeschlossen ist. Der erste Abschnitt 84 der Blutableitung führt zu einer Tropfkammer 78, von welcher das Blut über den zweiten Abschnitt 85 der Blutableitung zurück zum Patienten geführt wird.

Von der zweiten Kammer 68 des zweiten Sterilfilters 60 geht die Substituatleitung 72 ab, in die eine Substituatpumpe 76 eingeschaltet ist, und führt ebenfalls zur Tropfkammer 78.

Von der Dialysierflüssigkeitsquelle 26 wird frische Dialysierflüssigkeit durch den ersten Zuleitungsabschnitt 28 der ersten Kammer 32 der Bilanzierungvorrichtung 30 zugeführt. Diese erste Bilanziereinheit 32 ist an die Zuleitung 22 des Dialysierflüssigkeitsweges 20 angeschlossen, so daß die frische Dialysierflüssigkeit von der ersten Bilanziereinheit 32 durch den zweiten Zuleitungsabschnitt 36, den Sterilfilter 38, den dritten Zuleitungsabschnitt 56, die erste Kammer 64 des zweiten Sterilfilter 60 und den vierten Zuleitungsabschnitt 70 zur ersten Kammer 14 des Dialysators 12 geleitet wird. Beim Durchgang durch die Membran 40 des ersten Sterilfilters 38 erfolgt eine sterilisierend wirkende Filtration der Dialysierflüssigkeit, so daß eine vollständig sterilisierte Dialysierflüssigkeit in die erste Kammer 14 des Dialysators 12 eingeleitet wird. Der Ausgang der ersten Kammer 14 des Dialysators 12 ist an die Ableitung 24 angeschlossen, welche zu der zweiten Bilanziereinheit 34 der Bilanzierungsvorrichtung 30 führt. In diese Ableitung 24 ist eine Dialysierflüssigkeitspumpe 86 angeordnet, welche die Dialysierflüssigkeit in dem Dialysierflüssigkeitsweg 20 von der ersten Bilanziereinheit 32 zur zweiten Bilanziereinheit 34 der Bilanzierungsvorrichtung 30 fördert. Die Bilanzierungsvorrichtung 30 ist dabei so ausgebildet, daß eine durch die zweite Bilanziereinheit 34 in den Abfluß 90 strömende Menge an verbrauchter Dialysierflüssigkeit durch eine gleiche Menge frischer Dialysierflüssigkeit ersetzt wird, die durch die erste Bilanziereinheit 32 in die Zuleitung 22 gefördert wird. In den vierten Zuleitungsabschnitt 70 ist ein Absperrorgan 71 in Form einer regelbaren Klemme geschaltet, mit dem dieser Leitungsabschnitt 70 im Bedarfsfall abgesperrt werden kann. Dies hat zur Folge, daß die Vorrichtung 10 dann ausschließlich als Hämofiltrationsanordnung arbeitet.

Im Falle der Hämofiltration wird dann vorzugsweise anstelle eines Dialysators 12 ein Hämofilter eingesetzt.

Gemäß einer Ausführungsform kann die Klemme 71 so betätigt werden, daß sie den Querschnitt der Leitung 70 schrittweise verengt oder erweitert. Dies hat zur Folge, daß unterschiedliche Fluidmengen durchgelassen werden, so daß der Fluidfluß durch die Membran 62 in entsprechender Weise fällt oder steigt, beispielsweise kann dies in Abstimmung mit der Pumpe 76 erfolgen.

Die durch die frische Dialysierflüssigkeit in die Zuleitung 22 eingeschleppten Keime und Pyrogene werden durch die Membran 40 des ersten Sterilfilters 38 gefiltert und setzen sich an dieser ab. Durch Öffnen des Bypassventils 54 in der Bypassleitung 52, die eine Verbindung von der ersten Kammer 42 zur Ableitung 24 herstellt, erfolgt eine Durchspülung der ersten Kammer 42 mit Dialysierflüssigkeit. Dieses bewirkt, daß die an der Membran 40 abgesetzten Partikel, Keime und Pyrogene durch die an dieser vorbeiströmenden Dialysierflüssigkeit mitgerissen werden und über die Bypassleitung 52, die Ableitung 24 durch die zweit Bilanziereinheit 34 hindurch in den Abfluß 90 gespült werden. Damit ist ein wirkungsvolles Freispülen der Membran 40 des ersten Sterilfilters 38 möglich, so daß die Gefahr einer Ruptur erheblich verringert ist, die Sterilität der Dialysierflüssigkeit gewährleistet ist und diese Anordnung eine hohe Betriebssicherheit aufweist.

Durch Einschalten der Substituatpumpe 76 wird Dialysierflüssigkeit, welche durch die erste Kammer 64 strömt, durch die Membran 62 hindurch in die zweite Kammer 68 gezogen und gelangt von dort in die Substituatleitung 72. Diese Substituatleitung 72 ist zu einer im Blutweg 80 angeordneten Tropfkammer 78 geführt. In diese Tropfkamner 78 mündet ebenfalls ein erster Blutleitungsabschnitt 84, der mit einem Ende an den Ausgang der zweiten Kammer 16 des Dialysators 12 angeschlossen ist und mit dem anderen Ende in die Tropfkammer 78 mündet. Von der Tropfkammer 78 führt dann ein zweiter Blutableitungsabschnitt 85 zum Patienten. Es versteht sich von selbst, daß außer der hier dargestellten Postdilution auch eine Prädilution möglich ist.

Zusätzlich kann durch Betätigen der Ultrafiltratpumpe 88 Flüssigkeit aus dem hydraulisch geschlossenen Dialysierflüssigkeitsweg entfernt werden, welche dann durch die Membran 18 des Dialysators 12 hindurch aus dem durch die zweite Kammer 16 strömenden Blut nachgezogen wird.

Durch diese Anordnung wird zum einen eine doppelte Sicherheit gegen Kontamination der Substituatlösung erreicht, zum anderen wird durch das Freispülen sowohl der Membran 40 des ersten Sterilfilters 38 als auch der Membran 62 des zweiten Sterilfilters 60 eine Anhäufang von Keimen oder Pyrogenen verhindert, so daß die Gefahr einer Ruptur an einer dieser Membranen erheblich reduziert ist. Damit ist gewährleistet, daß dem Patienten stets eine völlig sterile Substituatlösung zugeführt wird.

### Bezugszeichenliste

- 10: Hämodiafiltrationsvorrichtung
- 12: Dialysator
- 14: erste Kammer
- 16: zweite Kammer
- 18: Membran
- 20: Dialysierflüssigkeitsweg
- 22: Zuleitung
- 24: Ableitung
- 25: Ultrafiltratleitung
- 26: Dialysierflüssigkeitsquelle
- 28: erster Zuleitungsabschnitt
- 30: Bilanzierungsvorrichtung
- 32: erste Bilanziereinheit
- 34: zweite Bilanziereinheit
- 36: zweiter Zuleitungsabschnitt
- 38: erster Sterilfilter
- 40: Membran
- 42: erste Kammer
- 44: zweite Kammer
- 52: Bypassleitung
- 54: Bypassventil
- 56: dritter Zuleitungsabschnitt
- 58: Ventil
- 60: zweites Sterilfilter
- 62: Membran
- 64: erste Kammer
- 68: zweite Kammer
- 70: vierter Zuleitungsabschnitt
- 71: Klemme
- 72: Substituatleilung
- 73: erster Substituatleitungsabschnitt
- 75: zweiter Substituatleitungsabschnitt
- 76: Substituatpumpe
- 78: Tropfkammer
- 80: Blutweg
- 82: Blutzuleitung
- 84: erster Blutableitungsabschnitt
- 85: zweiter Blutableitungsabschnitt
- 86: Dialysierflüssigkeitspumpe
- 88: Ultrafiltrationspumpe
- 90: Abfluß

## Patentansprüche

1. Hämofiltrationsvorrichtung oder Hämodiafiltrationsvorrichtung mit einem Hämofilter bzw. Dialysator (12), der durch eine Membran in zwei Kammern (14, 16) geteilt ist, wobei die erste Kammer (14) Teil eines Dialysierflüssigkeitsweges (20) und die zweite Kammer (16) Teil eines Blutweges (80) ist, der Dialysierflüssigkeitsweg eine Zuleitung (22), die sich von einer Einrichtung zur Bereitstellung von Dialysierflüssigkeit (26) bis zum Hämofilter bzw. Dialysator erstreckt und in die eine erste Bilanziereinheit (32) geschaltet ist, und eine Ableitung (24) aufweist, die sich vom Hämofilter bzw. Dialysator zu einem Abfluß (90) erstreckt und in die eine zweite Bilanziereinheit (34) geschaltet ist, mit einer Pumpe (86) zum Fördern der Dialysierflüssigkeit im geschlossenen Dialysierflüssigkeitssystem, einer die Zuleitung mit der Ableitung des Dialysierflüssigkeitsweges verbindenden Bypassleitung (52), in die ein Bypassventil (54) geschaltet ist, einer Ultrafiltrationseinrichtung (25, 88), einem in der Zuleitung zwischen der ersten Bilanziereinheit und dem Hämofilter bzw. Dialysator angeordneten ersten Sterilfilter (38), der durch eine Keime zurückhaltende Membran in eine erste Kammer (42) und eine zweite Kammer (44) geteilt ist, einer von der Zuleitung zwischen der ersten Bilanziereinheit und dem Hämofilter bzw. Dialysator abgehenden Verbindungsleitung (72), die mit dem Blutweg verbunden ist und in die eine Pumpe (76) und mindestens ein zweiter Sterilfilter (60), der durch eine Keime zurückhaltende Membran in eine erste Kammer (64) und eine zweite Kammer (68) geteilt ist, geschaltet sind,
**dadurch gekennzeichnet,**
**daß** die erste Kammer (64) des zweiten Sterilfilters (60) in Dialysierflüssigkeits-Durchfluß ist, wobei der Auslaß der ersten Kammer (64) des zweiten Sterilfilters (60) mit dem Eingang der ersten Kammer (14) des Hämofilters bzw. Dialysators (12) verbunden ist.

2. Hämofiltrationsvorrichtung oder Hämodiafiltrationsvorrichtung nach Anspruch 1, **dadurch gekennzeichnet, daß** der erste Sterilfilter (38) in die Zuleitung (22) geschaltet ist und die erste Kammer (42) des ersten Sterilfilters (38) zumindest zeitweise in Durchfluß schaltbar ist.

3. Hämofiltrationsvorrichtung oder Hämodiafiltrationsvorrichtung nach Anspruch 2, **dadurch gekennzeichnet, daß** der Auslaß der ersten Kammer (42) des ersten Sterilfilters (38) mit der zur Ableitung (24) führenden Bypass-Leitung (52) verbunden ist.

4. Hämofiltrationsvorrichtung oder Hämodiafiltrationsvorrichtung nach Anspruch 1, **dadurch gekennzeichnet, daß** in den Abschnitt (70) der Zuleitung (22), der den Auslaß der ersten Kammer (64) des zweiten Sterilfilters (60) mit dem Eingang der ersten Kammer (14) des Hämofilters bzw. Dialysators (12) verbindet, ein erstes Absperrorgan (71) geschaltet ist, das vorbestimint den Querschnitt des Zuleitungsabschnitts (70) verengt oder unterbricht.

5. Hämofiltrationsvorrichtung oder Hämodiafiltrationsvorrichtung nach Anspruch 1, **dadurch gekennzeichnet, daß** in die Zuleitung (22) stromab des ersten Sterilfilters (38) ein Absperrorgan (58) geschaltet ist.

## Claims

1. A haemofiltration apparatus or haemodiafiltration apparatus with a haemofilter or dialyser (12) which is divided by a membrane into two chambers (14, 16), whereby the first chamber (14) is part of a dialysing fluid path (20) and the second chamber (16) part of a blood path (80), the dialysing fluid path comprises a feed line (22) which extends from a device for preparing dialysing fluid (26) to the haemofilter or dialyser and into which a first balancing unit (32) is incorporated, and a discharge line (24) which extends from the haemofilter or dialyser to a discharge (90) and into which a second a balancing unit (34) is incorporated, with a pump (86) for delivering dialysing fluid in the closed dialysing fluid system, a bypass line (52) connecting the feed line to the discharge line of the dialysing fluid path and into which a bypass valve (54) is connected, an ultra-filtration device (25, 88), a first sterile filter (38) disposed in the feed line between the first balancing unit and the haemofilter or dialyser and which is divided into a first chamber (42) and a second chamber (44) by a membrane which traps the germs, a connecting line (72) extending from the feed line between the first balancing unit and the haemofilter or dialyser and which is connected to the blood path and into which a pump (76) and at least one second sterile filter (60) are incorporated, the second sterile filter (60) being divided into a first chamber (64) and a second chamber (68) by a membrane which traps the germs, **characterised in that** the first chamber (64) of the second sterile filter (60) is connected into the dialysing fluid through-flow, whereby the outlet of the first chamber (64) of the second sterile filter (60) is connected to the inlet of the first chamber (14) of the haemofilter or dialyser (12).

2. A haemofiltration apparatus or haemodiafiltration apparatus according to claim 1, **characterised in that** the first sterile filter (38) is connected into the feed line (22) and the first chamber (42) of the first sterile filter (38) can be connected at least temporarily into the through-flow.

3. A haemofiltration apparatus or haemodiafiltration apparatus according to claim 2, **characterised in that** the outlet of the first chamber (42) of the first sterile filter (38) is connected to the bypass line (52) leading to the discharge line (24).

4. A haemofiltration apparatus or haemodiafiltration apparatus according to claim 1, **characterised in that** a first shut-off element (71) is incorporated into the section (70) of the feed line (22) which connects the outlet of the first chamber (64) of the second sterile filter (60) to the inlet of the first chamber (14) of the haemofilter or dialyser (12), said first shut-off element (71) constricting or interrupting the cross-section of the feed-line section (70) in a predetermined manner.

5. A haemofiltration apparatus or haemodiafiltration apparatus according to claim 1, **characterised in that** a shut-off element (58) is incorporated into the feed line (22) downstream of the first sterile filter (38).

## Revendications

1. Dispositif d'hémofiltration ou dispositif d'hémodiafiltration avec un hémofiltre ou un dialyseur (12) séparé par une membrane en deux chambres (14, 16), la première chambre (14) faisant partie d'une voie réservée au liquide de dialyse (20) et la deuxième chambre (16) faisant partie d'une voie réservée au sang (80), la voie réservée au liquide de dialyse comportant une conduite d'amenée (22) qui s'étend d'un dispositif de fourniture du liquide de dialyse (26) à l'hémofiltre ou au dialyseur et dans laquelle est placée une première unité d'équilibrage (32), et une conduite d'évacuation (24) qui s'étend de l'hémofiltre ou du dialyseur à un point d'évacuation (90) et dans laquelle est placée une deuxième unité d'équilibrage (34), avec une pompe (86) pour refouler le liquide de dialyse dans le système fermé de liquide de dialyse, une conduite de dérivation (52) qui relie les conduites d'amenée et d'évacuation de la voie réservée au liquide de dialyse et dans laquelle est placée une soupape de dérivation (54), un dispositif d'ultrafiltration (25, 88), un premier filtre stérile (38) disposé dans la conduite d'amenée entre la première unité d'équilibrage et l'hémofiltre ou le dialyseur et séparé par une membrane retenant les germes en une première chambre (42) et une deuxième chambre (44), une conduite de raccordement (72) qui part de la conduite d'amenée entre la première unité d'équilibrage et 1'hémofiltre ou le dialyseur et est reliée à la voie réservée au sang et dans laquelle sont placés une pompe (76) et au moins un deuxième filtre stérile (60) séparé par une membrane retenant les germes en une première chambre (64) et une deuxième chambre (68), **caractérisé en ce que** la première chambre (64) du deuxième filtre stérile (60) est insérée dans le circuit d'écoulement du liquide de dialyse, la sortie de la première chambre (64) du deuxième filtre stérile (60) étant reliée à l'entrée de la première chambre (14) de l'hémofiltre ou du dialyseur (12).

2. Dispositif d'hémofiltration ou dispositif d'hémodiafiltration selon la revendication 1, **caractérisé en ce que** le premier filtre stérile (38) est inséré dans la conduite d'amenée (22), et **en ce que** la première chambre (42) du premier filtre stérile (38) peut être insérée dans le circuit d'écoulement, au moins temporairement.

3. Dispositif d'hémofiltration ou dispositif d'hémodiafiltration selon la revendication 2, **caractérisé en ce que** la sortie de la première chambre (42) du premier filtre stérile (38) est reliée à la conduite de dérivation (52) qui mène à la conduite d'évacuation (24).

4. Dispositif d'hémofiltration ou dispositif d'hémodiafiltration selon la revendication 1, **caractérisé en ce qu'**est inséré dans le tronçon (70) de la conduite d'amenée (22), raccordant la sortie de la première chambre (64) du deuxième filtre stérile (60) à l'entrée de la première chambre (14) de l'hémofiltre ou du dialyseur (12), un premier organe d'arrêt (71), qui rétrécit ou condamne de manière prédéterminée la section du tronçon de conduite d'amenée (70).

5. Dispositif d'hémofiltration ou dispositif d'hémodiafiltration selon la revendication 1, **caractérisé en ce qu'**est inséré dans la conduite d'amenée (22) en aval du premier filtre stérile (38), un organe d'arrêt (58).
